# EUROPEAN PATENT APPLICATION

(11) **EP 4 070 743 A1**
(43) Date of publication of application: **12.10.2022**
(21) Application number: 22166412.1
(22) Date of filing: 01.04.2022
(51) Int. Cl.: A61B 17/22

(54) **LITHOTRIPSY PROBE ASSEMBLIES AND LITHOTRIPSY SYSTEMS**

(30) Priority: 05.04.2021 US 202163170786 P
(71) Applicant: Cook Medical Technologies LLC, Bloomington, IN 47404 (US)
(72) Inventor: OBERMILLER, Joseph, West Lafayette, 47906 (US); JONES, Matthew, West Lafayette, 47906 (US); DOWELL, Angela, Lafayette, 47904 (US); BHAGWAT, Apoorva, Irvine, 92618 (US); PUCKETT, Daniel, Dallas, 75205 (US); FERRARI, Eugene, West Lafayette, 47906 (US)
(74) Representative: Leach, Sean Adam

(57) **Abstract**

Example lithotripsy probe assemblies, lithotripsy systems, and methods of using a lithotripsy system are described. An example lithotripsy probe assembly has a probe coupling, a probe, and a plurality of sealing members disposed on the probe coupling. The probe coupling has a probe coupling proximal end, a probe coupling distal end, a probe coupling length, and a probe coupling main body that defines a plurality of projections, a probe coupling first passageway, and a probe coupling second passageway. The probe coupling length extends from the probe coupling proximal end to the probe coupling distal end. The probe is attached to the probe coupling and is partially disposed within the probe coupling first passageway. The probe has a probe proximal end, a probe distal end, and a probe length that extends from the probe proximal end to the probe distal end and is greater than the probe coupling length.

## Description

### Related Application

This application claims priority to U.S. Provisional Application No. 63/170,786, filed April 5, 2021. The entire contents of this related application are hereby incorporated by reference into this disclosure.

### Field

The disclosure relates generally to the field of medical devices. More particularly, the disclosure relates to lithotripsy probe assemblies, lithotripsy systems, and methods of using a lithotripsy system.

### Background

It is sometimes necessary, or otherwise desirable, to remove unwanted materials disposed within a bodily passage. For example, lithotripsy - the disruption and removal of calculi, or stones, from a region of the body - is frequently performed to remove stones disposed in a salivary duct or the urinary tract.

Various types of lithotripsy are known, including shockwave lithotripsy, extracorporeal shockwave lithotripsy, laser lithotripsy, percutaneous lithotripsy, endoscopic lithotripsy, pneumatic lithotripsy, ultrasonic lithotripsy, and electrokinetic lithotripsy. Generally, a probe is attached to a generator and is advanced through a previously placed sheath, or the working channel of a scope, until the distal end of the probe contacts a stone disposed in a bodily passage. In treatments in which an electrokinetic lithotripter is being utilized, the generator is activated and produces an impact between the distal end of the probe and the stone causing fragmentation of the stone and enabling its removal, which is accomplished through the application of a suction force to a lumen defined by the probe. However, performing lithotripsy in this manner can be challenging in relatively small body passages, such as the salivary ducts and urinary tract, due to the rigidity of the probes. Laser fibers can be used to accommodate these smaller bodily passages and to address these setbacks. However, laser fibers can require prolonged periods of time to fragment stones, do not provide suction during stone fragmentation, and many scopes used with laser fibers are limited in their ability to access various portions of the body, such as the lower pole of the kidney.

A need exists, therefore, for new and improved lithotripsy probe assemblies, lithotripsy systems, and associated methods.

### Summary of Selected Example Embodiments

Various example lithotripsy probe assemblies, lithotripsy systems, and methods of using a lithotripsy system are described herein.

An example lithotripsy probe assembly has a probe coupling and a probe. The probe coupling has a probe coupling lengthwise axis, a probe coupling proximal end, a probe coupling distal end, a probe coupling length, and a probe coupling main body that defines a probe coupling side wall, a plurality of projections, a probe coupling first passageway, and a probe coupling second passageway. The probe coupling length extends from the probe coupling proximal end to the probe coupling distal end. Each projection of the plurality of projections extends from the probe coupling side wall and away from the probe coupling lengthwise axis. The probe coupling first passageway extends from the probe coupling proximal end to the probe coupling distal end. The probe coupling second passageway extends through the probe coupling side wall and is in fluid communication with the probe coupling first passageway. The probe is attached to the probe coupling and is partially disposed within the probe coupling first passageway. The probe has a probe proximal end, a probe distal end, a probe length, and a probe main body that defines a probe distal tip. The probe length extends from the probe proximal end to the probe distal end. The probe length is greater than the probe coupling length.

Another example lithotripsy probe assembly has a probe coupling, a probe, a plurality of sealing members, a spring, and a housing. The probe coupling has a probe coupling lengthwise axis, a probe coupling proximal end, a probe coupling distal end, a probe coupling length, and a probe coupling main body that defines a probe coupling side wall, a plurality of projections, a probe coupling first passageway, and a probe coupling second passageway. The probe coupling length extends from the probe coupling proximal end to the probe coupling distal end. Each projection of the plurality of projections extends from the probe coupling side wall and away from the probe coupling lengthwise axis. The probe coupling first passageway extends from the probe coupling proximal end to the probe coupling distal end. The probe coupling second passageway extends through the probe coupling side wall and is in fluid communication with the probe coupling first passageway. The probe is attached to the probe coupling and is partially disposed within the probe coupling first passageway. The probe has a probe proximal end, a probe distal end, a probe length, and a probe main body that defines a probe distal tip. The probe length extends from the probe proximal end to the probe distal end. The probe length is greater than the probe coupling length. The plurality of sealing members is disposed on the probe coupling. A sealing member of the plurality of sealing members is disposed between the plurality of projections and the probe coupling distal end. The spring is disposed on the probe coupling and is disposed adjacent to the sealing member of the plurality of sealing members. The housing is disposed on the probe, the probe coupling, and the spring. The housing has a housing proximal end, a housing distal end, and a housing main body that defines a housing side wall, a housing side port, a housing first passageway, and a housing second passageway. The housing first passageway extends from the housing proximal end to the housing distal end. The housing second passageway extends through the housing side port and is in fluid communication with the housing first passageway and the probe coupling second passageway.

Another example lithotripsy probe assembly has a probe coupling, a probe, a plurality of sealing members, a spring, a housing, and a scope coupling. The probe coupling has a probe coupling lengthwise axis, a probe coupling proximal end, a probe coupling distal end, a probe coupling length, and a probe coupling main body that defines a probe coupling side wall, a plurality of projections, a probe coupling first passageway, and a probe coupling second passageway. The probe coupling length extends from the probe coupling proximal end to the probe coupling distal end. Each projection of the plurality of projections extends from the probe coupling side wall and away from the probe coupling lengthwise axis. The probe coupling first passageway extends from the probe coupling proximal end to the probe coupling distal end. The probe coupling second passageway extends through the probe coupling side wall and is in fluid communication with the probe coupling first passageway. The probe is attached to the probe coupling and is partially disposed within the probe coupling first passageway. The probe has a probe proximal end, a probe distal end, a probe length, and a probe main body that defines a probe distal tip. The probe length extends from the probe proximal end to the probe distal end. The probe length is greater than the probe coupling length. The plurality of sealing members is disposed on the probe coupling. A sealing member of the plurality of sealing members is disposed between the plurality of projections and the probe coupling distal end. The spring is disposed on the probe coupling and contacts the sealing member of the plurality of sealing members. The housing is disposed on the probe, the probe coupling, and the spring. The housing has a housing proximal end, a housing distal end, and a housing main body that defines a housing side wall, a housing side port, a housing first passageway, and a housing second passageway. The housing first passageway extends from the housing proximal end to the housing distal end. The housing second passageway extends through the housing side port and is in fluid communication with the housing first passageway and the probe coupling second passageway. The scope coupling is releasably attached to the housing and is disposed adjacent to the spring.

An example lithotripsy system includes a lithotripsy probe assembly, a scope assembly, a generator, and a suction device. The lithotripsy probe assembly has a probe coupling, a probe, a plurality of sealing members, a spring, a housing, and a scope coupling. The scope assembly is releasably attached to the scope coupling. The generator is releasably attached to the scope coupling.

An example method of using a lithotripsy system comprises: advancing a spring over a probe and a probe coupling such that the spring contacts a sealing member; attaching a scope coupling to a housing; advancing the probe, the probe coupling, and the spring into a first passageway of the housing such that the spring contacts the scope coupling to create a lithotripsy probe assembly; attaching a first end of a tube to a side port of the housing; attaching a generator to the housing; introducing a scope into a bodily passage; advancing the scope to a stone disposed within the bodily passage; introducing the probe into a working channel of the scope such that the probe is partially disposed within the working channel and extends distal to the distal end of the scope; attaching the scope coupling to the scope such that a first passageway of the probe coupling is in fluid communication with the working channel of the scope; contacting the probe to the stone; activating the generator such that the probe fragments the stone; applying suction to the tube such that material disposed outside the working channel of the scope is drawn into the working channel of the scope, through a passageway defined by the scope coupling, through a first passageway of the probe coupling, through a second passageway of the probe coupling, through the side port of the housing, and through the tube; deactivating the generator; withdrawing the probe and the scope from the bodily passage.

Additional understanding of these example lithotripsy probe assemblies, lithotripsy systems, and methods can be obtained by review of the detailed description, below, and the appended drawings.

### Brief Description of the Drawings

FIG. 1 is a partial elevation view of an example lithotripsy probe assembly.
FIG. 2 is a partial exploded view of the lithotripsy probe assembly illustrated in FIG. 1.
FIG. 3 is a partial elevation view of a proximal portion of the lithotripsy probe assembly illustrated in FIG. 1. The probe and spring are partially disposed within the housing.
FIG. 4 is an elevation view of the housing and the scope coupling of the lithotripsy probe assembly illustrated in FIG. 1.
FIG. 5 is a cross-sectional view of the housing of the lithotripsy probe assembly illustrated in FIG. 1 taken along the lengthwise axis of the housing.
FIG. 6 is a partial elevation view of the probe, the probe coupling, the plurality of sealing members, and the spring of the lithotripsy probe assembly illustrated in FIG. 1.
FIG. 7 is a partial perspective view of the probe, the probe coupling, and the plurality of sealing members of the lithotripsy probe assembly illustrated in FIG. 1.
FIG. 8 is a partial top view of the probe, the probe coupling, and the plurality of sealing members of the lithotripsy probe assembly illustrated in FIG. 1.
FIG. 9 is a sectional view of the probe and probe coupling of the lithotripsy probe assembly illustrated in FIG. 1 taken along the lengthwise axis of the probe coupling.
FIG. 10 is a partial elevation view of an example probe distal tip.
FIG. 11 is a partial elevation view of another example probe distal tip.
FIG. 12 is a partial elevation view of another example probe distal tip.
FIG. 13 is a partial elevation view of another example probe distal tip.
FIG. 14 is a partial elevation view of another example probe distal tip.
FIG. 15 is a partial elevation view of an example lithotripsy system.
FIG. 16 is a partial elevation view of the lithotripsy probe assembly of the lithotripsy system illustrated in FIG. 15 free of the scope and the generator.
FIG. 17 is another partial elevation view of the lithotripsy system illustrated in FIG 15. The lithotripsy system is illustrated free of the scope and attached to a suction device.
FIG. 18 is a partial perspective view of another example lithotripsy system.
FIG. 19 is a schematic illustration of an example method of using a lithotripsy system.

### Detailed Description of Selected Examples

The following detailed description and the appended drawings describe and illustrate various example lithotripsy probe assemblies, lithotripsy systems, and methods of using a lithotripsy system. The description and illustration of these examples are provided to enable one skilled in the art to make and use a lithotripsy probe assembly, a lithotripsy system, and to practice a method of using a lithotripsy system. They are not intended to limit the scope of the invention, or the protection sought, in any manner. The invention is capable of being practiced or carried out in various ways and the examples described and illustrated herein are merely selected examples of the various ways of practicing or carrying out the invention and are not considered exhaustive.

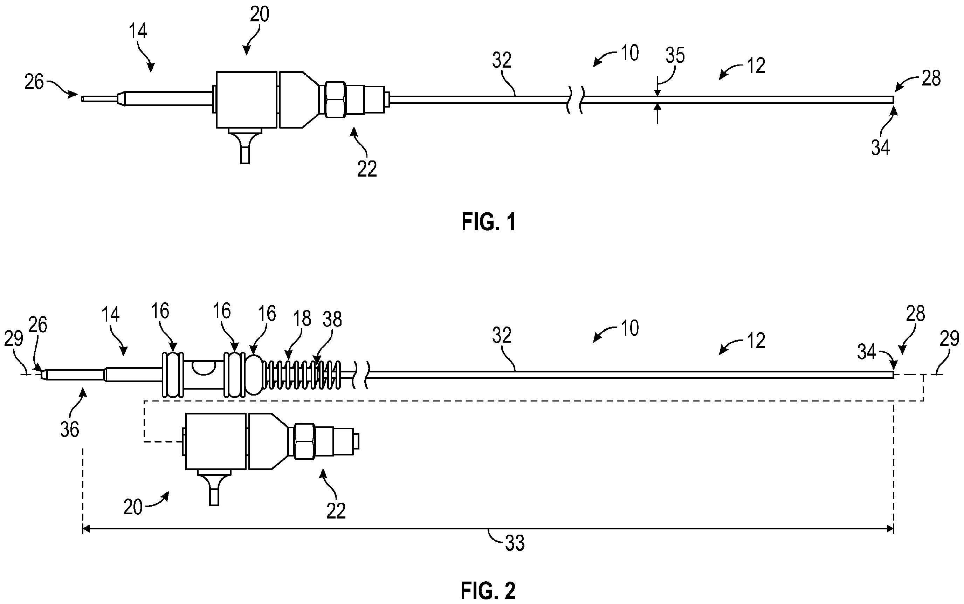

FIGS. 1, 2, 3, 4, 5, 6, 7, 8, and 9 illustrate a first example lithotripsy probe assembly 10 that includes a probe 12, a probe coupling 14, a plurality of sealing members 16, a spring 18, a housing 20, and a scope coupling 22.

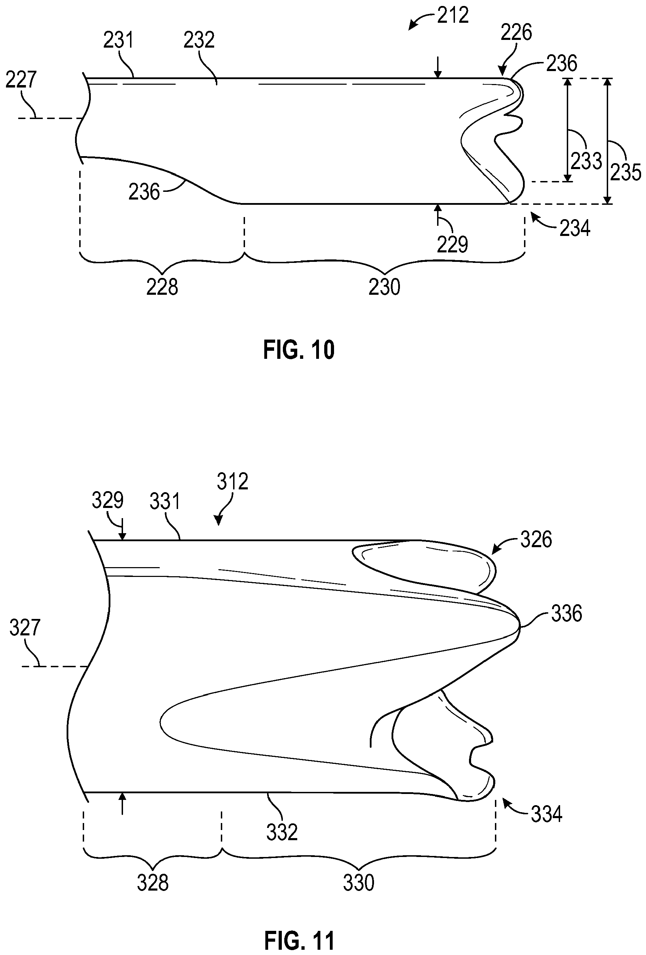

In the illustrated embodiment, the probe 12 has a proximal end 26, a distal end 28, a lengthwise axis 29, a proximal portion 30 that extends from the proximal end 26 toward the distal end 28, and a main body 32 that defines a distal tip 34. The probe 12 is attached to the probe coupling 14 and has a length 33 and an outside diameter 35 that is constant along its length 33. However, alternative embodiments can include a probe that has an outside diameter that varies along its length. The probe 12 is partially disposed within the probe coupling 14 such that the proximal end 26 and the proximal portion 30 are disposed within the first passageway 46 defined by the probe coupling 14, as described in more detail herein, and the distal end 28 is disposed outside of, and distal to, the first passageway 46 defined by the probe coupling 14. However, alternative embodiments can include a probe that has a proximal end and/or proximal portion disposed outside of, or proximal to, a lumen defined by a probe coupling and/or a proximal portion that is disposed between the proximal end and distal end of a probe. The distal tip 34 is configured to fragment stones disposed within a bodily passage such that they can be removed from the bodily passage and can have any suitable structural arrangement. Examples of structural arrangements considered suitable for a distal tip are described in more detail herein with respect to FIGS. 10, 11, 12, 13, and 14.

The probe coupling 14 is attached to a proximal portion 30 of the probe 12 such that the probe 12 is partially disposed within the probe coupling 14. In the illustrated embodiment, the probe coupling 14 has a proximal end 36, a distal end 38, a length 37, a lengthwise axis 39, and a main body 40 that defines a side wall 42, a plurality of projections 44, a first passageway 46, and a second passageway 48. The length 37 of the probe coupling 14 is less than the length 33 of the probe 12. The side wall 42 has a proximal portion 50, a first intermediate portion 52, a second intermediate portion 54, and a distal portion 56. The proximal portion 50 has a first outside diameter 51, the first intermediate portion 52 has a second outside diameter 53, the second intermediate portion 54 has a third outside diameter 55, and the distal portion 56 has a fourth outside diameter 57. The second outside diameter 53 is greater than the first outside diameter 51, the third outside diameter 55 is greater than the second outside diameter 53, and the fourth outside diameter 57 is less than the third outside diameter 55. The distal portion 56 has a length 59 that extends from the plurality of projections 44 to the distal end 38 of the probe coupling 14. Each projection of the plurality of projections 44 extends from the second intermediate portion 54 of the side wall 42 and away from the lengthwise axis 39 of the probe coupling 14. A first set of projections 58 of the plurality of projections 44 is disposed between the proximal end 36 of the probe coupling 14 and the second passageway 48. A second set of projections 60 of the plurality of projections 44 is disposed between the distal end 38 of the probe coupling 14 and the second passageway 48. Each projection of the plurality of projections 44 has an outside diameter 45 that is greater than the third outside diameter 55 of the second intermediate portion 54 of the side wall 42. The first passageway 46 extends from the proximal end 36 to the distal end 38 of the probe coupling 14 and has a first portion 62 and a second portion 64. The probe 12 is partially disposed within the first passageway 46. The first portion 62 of the first passageway 46 extends from the proximal end 36 toward the distal end 38 to a location proximal to the second passageway 48. The second portion 64 of the first passageway 46 extends from the first portion 62 to the distal end 38. The first portion 62 has a first inside diameter 63 that is less than the first outside diameter 51 of the proximal portion 50 of the side wall 42. The second portion 64 has a second inside diameter 65 that is greater than the first inside diameter 63, less than the fourth outside diameter 57 of the distal portion 56 of the side wall 42, and greater than the outside diameter 35 of the probe 12. This structural configuration provides a mechanism for fluid and fragments of stone to pass through the second portion 64 of the first passageway 46 between the probe coupling 14 and the probe 12 and through the second passageway 48 such that the fluid and fragments of stone can be removed from a bodily passage within which the probe 12 is disposed. The second passageway 48 extends through the side wall 42 of the probe coupling 14 between the first set of projections 58 and the second set of projections 60 and is in fluid communication with the first passageway 46. The second passageway 48 has an inside diameter 49 that is greater than the first inside diameter 63 of the first passageway 46.

A probe and probe coupling included in a lithotripsy probe assembly can be formed of any suitable material and have any suitable dimensions and selection of a suitable material to form a probe and probe coupling and suitable dimensions for a probe and probe coupling can be based on various considerations, including the intended use of the lithotripsy probe assembly of which the probe and probe coupling is a component. Examples of materials considered suitable to form a probe and a probe coupling include biocompatible materials, materials that can be made biocompatible, stainless steel, Nitinol, and any other material considered suitable for a particular embodiment. Examples of dimensions considered suitable to form a probe include probes that have an outside diameter that is equal to, about, greater than, or less than 0.025 inches, 0.027 inches, 0.031 inches, 0.037 inches, outside diameters between 0.02 inches and 0.04 inches, outside diameters greater than 0.02 inches, probes that have a length that is equal to, about, greater than, or less than 93.3 centimeters, 93.4 centimeters, 93.6 centimeters, 93.9 centimeters, lengths between 90 centimeters and 98 centimeters, lengths greater than 90 centimeters (e.g., when used with flexible ureteroscopes), lengths less than 50 centimeters (e.g., when used with sialendoscopes), and any other dimensions considered suitable for a particular embodiment.

Examples of dimensions considered suitable to form a probe coupling include probe couplings that have a first angled portion on a proximal end that has a length equal to, about, greater than, or less than 0.9 millimeters and is disposed at an angle equal to, about, greater than, or less than 20 degrees relative to a lengthwise axis of the probe coupling, a second angled portion between a proximal portion and a first intermediate portion that is disposed a distance from the proximal end equal to, about, greater than, or less than 18 millimeters and at an angle equal to, about, greater than, or less than 30 degrees relative to a lengthwise axis of the probe coupling, a first set of projections disposed a distance from a proximal end equal to, about, greater than, or less than 31.75 millimeters, a center of a second passageway disposed a distance from a proximal end equal to, about, greater than, or less than 37.7 millimeters, a first projection of a set of projections separated from a second projection of the set of projections by a distance equal to, about, greater than, or less than 2 millimeters, a projection that has a thickness equal to, about, greater than, or less than 0.5 millimeters, a first set of projections separated from a second set of projections by a distance equal to, about, greater than, or less than 6 millimeters, a first outside diameter equal to, about, greater than, or less than 2.9 millimeters, a first inside diameter equal to, about, greater than, or less than 0.86 millimeters, a second outside diameter equal to, about, greater than, or less than 3.5 millimeters, a third outside diameter equal to, about, greater than, or less than 6 millimeters, a fourth outside diameter equal to, about, greater than, or less than 3.5 millimeters, a projection that has an outside diameter equal to, about, greater than, or less than 8.6 millimeters, a distal portion that has a length equal to, about, greater than, or less than 15 millimeters, a distal portion disposed a distance from a proximal end equal to, about, greater than, or less than 63.7 millimeters, a wall thickness along a distal portion equal to, about, greater than, or less than 0.5 millimeters, and/or any other dimensions considered suitable for a particular embodiment. A probe coupling can be attached to a probe using any suitable method or technique and selection of a suitable method or technique to accomplish attachment of a probe coupling to a probe can be based on various considerations, including the material forming a probe. Examples of methods and techniques considered suitable to attach a probe coupling to a probe include press-fitting, using an adhesive, welding, crimping, and/or any other method or technique considered suitable for a particular embodiment.

The plurality of sealing members 16 is disposed on the probe coupling 14 such that a first sealing member 66 of the plurality of sealing members 16 is disposed between a first projection 68 and a second projection 70 of the first set of projections 58, a second sealing member 72 of the plurality of sealing members 16 is disposed between a third projection 74 and a fourth projection 76 of the second set of projections 60, and a third sealing member 78 of the plurality of sealing members 16 is disposed between the second set of projections 60 and the distal end 38 of the probe coupling 14. Each of the first sealing member 66 and the second sealing member 72 has an outside diameter 73 that is greater than the outside diameter 45 of the plurality of projections 44. The third sealing member 78 of the plurality of sealing members 16 has an outside diameter 79.

A sealing member included in a lithotripsy probe assembly can comprise any suitable feature, device, or component capable of sealing an area between a probe coupling and a housing (e.g., first and second sealing members 66, 72) such that suction can be applied to the passageways defined by the probe coupling while also allowing movement of a probe coupling relative to the housing and/or that provides a mechanism for assisting with resetting the position of a probe and probe coupling during use (e.g., third sealing member 78). Selection of a suitable sealing member to include in a lithotripsy probe assembly can be based on various considerations, including the intended use of the lithotripsy probe assembly. Examples of sealing members considered suitable to include in a lithotripsy probe assembly include o-rings, gaskets, forming a portion of a probe coupling as a flexible raised projection, and any other sealing member considered suitable for a particular embodiment.

The spring 18 is partially disposed over the probe coupling 14. However, alternative embodiments can include a spring that is entirely disposed over a probe coupling. In the illustrated embodiment, the spring 18 is partially disposed over the distal portion 56 of the probe coupling 14 and distal to the third sealing member 78 of the plurality of sealing members 16. The spring 18 has a proximal end 80, a distal end 82, a length 81, an outside diameter 83, and a main body 84 that defines a passageway 86 that extends from the proximal end 80 to the distal end 82. The proximal end 80 of the spring 18 is adjacent to and contacts the third sealing member 78 when the probe assembly is attached to a generator, as described in more detail herein. The length 81 of the spring 18 is greater than the length 59 of the distal portion 56 of the probe coupling 14. The outside diameter 83 of the spring 18 is less than the outside diameter 45 of the plurality of projections 44. The passageway 86 of the spring 18 has an inside diameter 87 that is less than the outside diameter 79 of the third sealing member 78 of the plurality of sealing members 16. The spring 18 provides a mechanism for assisting with resetting the position of a probe and probe coupling during use via its interaction with the third sealing member 78.

A spring included in a lithotripsy probe assembly can comprise any suitable type of spring capable of resisting a compression force between a sealing member and a scope coupling, as described in more detail herein. Selection of a suitable spring to include in a lithotripsy probe assembly can be based on various considerations, including the material that forms a sealing member and/or a scope coupling. Examples of springs considered suitable to include in a lithotripsy probe assembly include coil springs, compression springs, coil compression springs, springs that have an outside diameter of 0.24 inches, springs that have outside diameters between 0.20 inches and 0.30 inches, springs that have an inside diameter of 0.176 inches, spring that have inside diameters between 0.15 inches and 0.19 inches, springs that have lengths of 15 millimeters, springs that have lengths between 10 millimeters and 20 millimeters, springs that have a rating of 15.9 lb/in, springs that have ratings between 13 lb/in and 19 lb/in, and any other spring considered suitable for a particular embodiment.

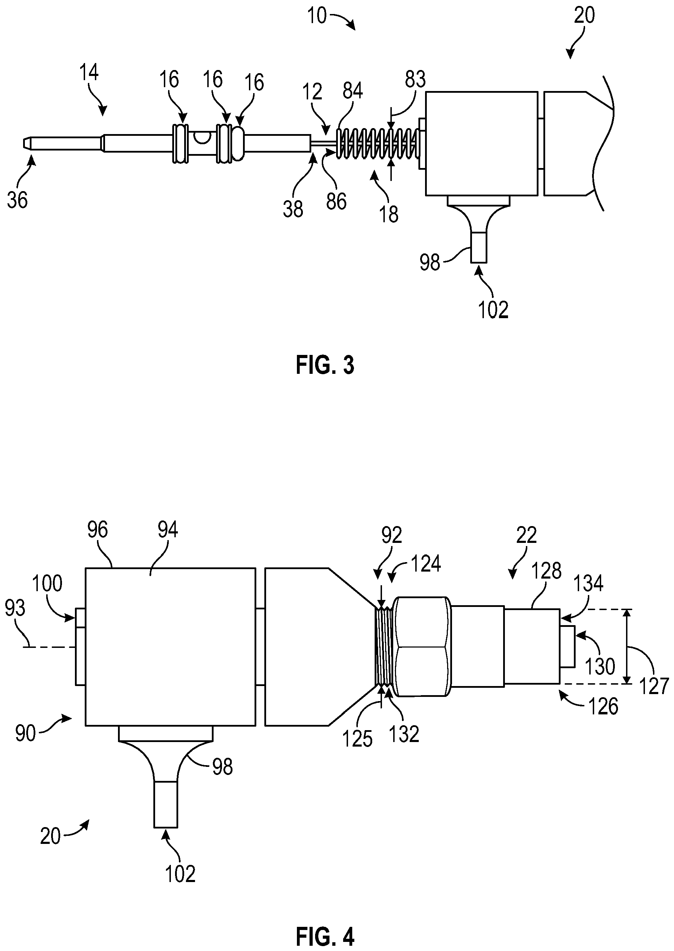

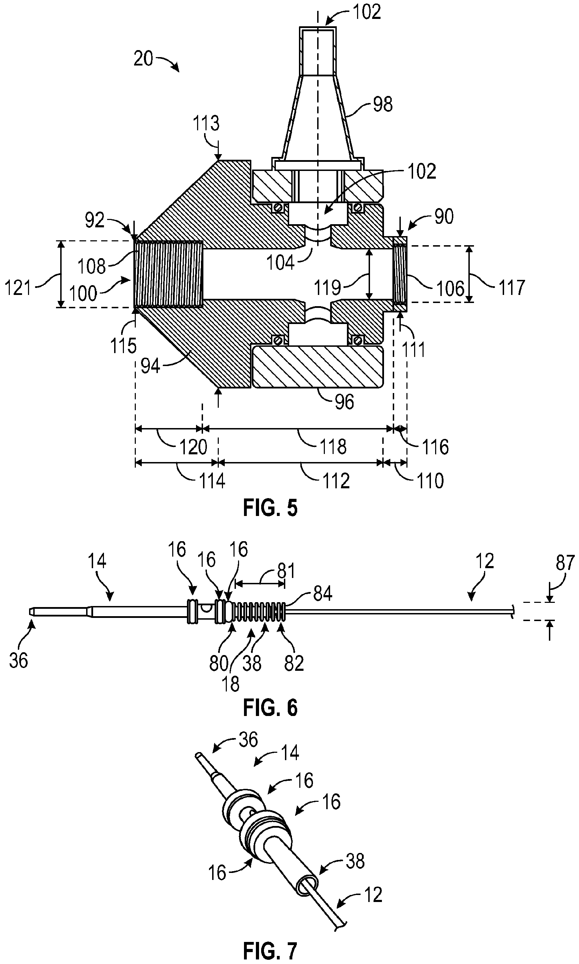

The housing 20 is disposed on the probe 12 and the probe coupling 14. In the illustrated embodiment, as shown in FIGS. 4 and 5, the housing 20 has a proximal end 90, a distal end 92, a lengthwise axis 93, and a main body 94 that defines a side wall 96, a side port 98, a first passageway 100, a second passageway 102, an opening 104, a first threaded portion 106, and a second threaded portion 108. The side wall 96 has a proximal portion 110, an intermediate portion 112, and a distal portion 114. The proximal portion 110 has a first outside diameter 111, the intermediate portion 112 has a second outside diameter 113, and the distal portion 114 has a third outside diameter 115. The second outside diameter 113 is greater than the first outside diameter 111 and the third outside diameter 115. The side port 98 extends from the intermediate portion 112 of the side wall 96 and away from the lengthwise axis 93 of the housing 20. The side port 98 is configured to be releasably attached to a suction device such that suction can be applied through the first and second passageways 46, 48 of the probe coupling 14, as described in more detail herein. The first passageway 100 extends from the proximal end 90 to the distal end 92 of the housing 20 and has a proximal portion 116, an intermediate portion 118, and a distal portion 120. The proximal portion 116 has a first inside diameter 117, the intermediate portion 118 has a second inside diameter 119, and the distal portion 120 has a third inside diameter 121. The second inside diameter 119 is less than the first inside diameter 117 and the third inside diameter 121. The second passageway 102 extends through the side port 98 and is in fluid communication with the first passageway 100 of the housing 20. The main body 94 defines the opening 104 between the first passageway 100 and the second passageway 102 such that the first and second passageways 100, 102 are in fluid communication with one another. Each of the probe 12, the probe coupling 14, and the spring 18 are disposed within the housing 20 (e.g., first passageway 100). The probe 12 is partially disposed within the first passageway of the housing 100. The probe coupling 14 is partially disposed within the first passageway 100 of the housing 20 such that the second passageway 48 of the probe coupling 14 is in fluid communication with the second passageway 102 of the housing 20. The first threaded portion 106 is defined within the proximal portion 116 of the first passageway 100 and is configured to mate with the threads of a generator, as described in more detail herein, such that the generator can be releasably attached to the housing 20. The second threaded portion 108 is defined within the distal portion 120 of the first passageway 100 and is configured to mate with the first threaded portion 132 of the scope coupling 22, as described in more detail herein, such that the scope coupling 22 can be releasably attached to the housing 20.

The scope coupling 22 is releasably attached to the distal end 92 of the housing 20 and is configured to be attached to a scope, as described in more detail herein. In the illustrated embodiment, as shown in FIG. 4, the scope coupling 22 has a proximal end 124, a distal end 126, and a main body 128 that defines a passageway 130, a first threaded portion 132, and a second threaded portion 134. The proximal end 124 of the scope coupling 22 has an outside diameter 125 and an inside diameter 127. The inside diameter 127 is less than the outside diameter 83 of the spring 18. The passageway 130 extends from the proximal end 124 to the distal end 126 and is in fluid communication with the first passageway 100 defined by the housing 20. The first threaded portion 132 is defined on the proximal end 124 and is configured to mate with the second threaded portion 108 of the housing 20 to accomplish releasable attachment between the housing 20 and scope coupling 22. The second threaded portion 134 is defined on the distal end 126 and is configured to mate with a threaded portion of a scope to accomplished releasable attachment between the scope coupling and the scope. When the lithotripsy probe assembly 10 is assembled, the spring 18 is disposed between the third sealing member 78 and the scope coupling 22 (e.g., within the first passageway 100 of the housing 20) such that the proximal end 80 of the spring contacts the third sealing member 78 and the distal end 82 of the spring 18 contacts the scope coupling 22.

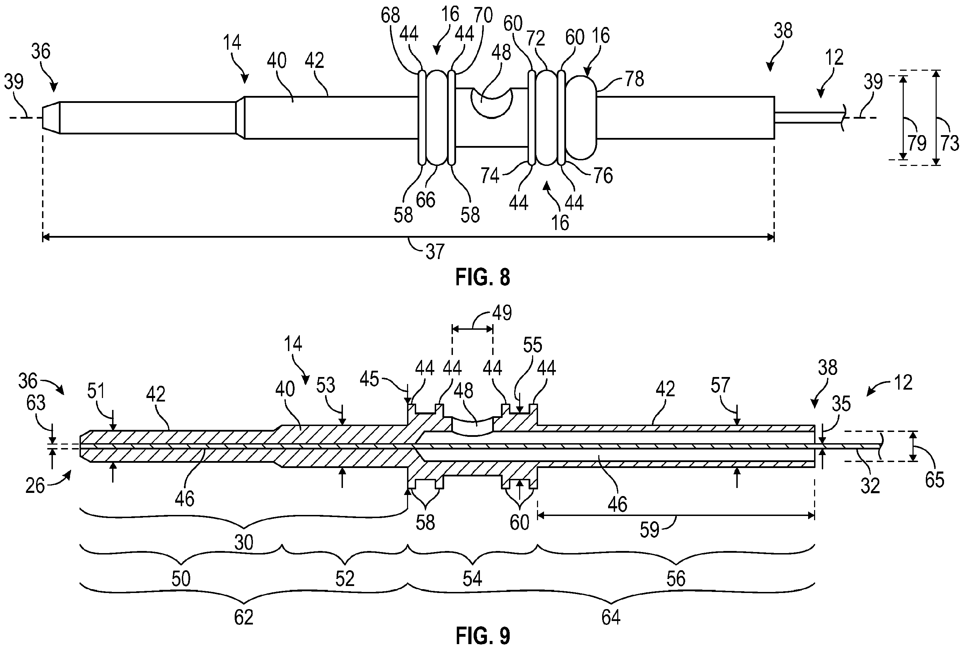

While the lithotripsy probe assembly 10 has been illustrated as including a probe 12, a probe coupling 14, a plurality of sealing members 16, a spring 18, a housing 20, and a scope coupling 22 an alternative embodiment can omit the inclusion of a plurality of sealing members, a spring, a housing, and/or a scope coupling. For example, an alternative embodiment of a lithotripsy probe assembly can be provided as illustrated in FIG. 8 such that it includes a probe, a probe coupling, and a plurality of sealing members or can be provided as illustrated in FIG. 9 such that it includes a probe and a probe coupling.

Each sealing member of a plurality of sealing members, a spring, a housing, and a scope coupling included in a lithotripsy probe assembly can be formed of any suitable material using any suitable method of manufacture and selection of a suitable material to form a sealing member, a spring, a housing, and a scope coupling can be based on various considerations, including the intended use of the lithotripsy probe assembly. Examples of materials considered suitable to form a sealing member, a spring, a housing, and/or a scope coupling include biocompatible materials, materials that can be made biocompatible, metals, stainless steel, Nitinol, polymers, and any other material considered suitable for a particular embodiment. While the probe 12, the probe coupling 14, the plurality of sealing members 16, the spring 18, the housing 20, and the scope coupling 22 have been illustrated as having a particular structural arrangement, a probe, a probe coupling, a sealing member, a spring, a housing, and a scope coupling can have any suitable structural arrangement.

FIG. 10 illustrates an example of an alternative probe 212 that can be included in a lithotripsy probe assembly. In the illustrated embodiment, the probe 212 has a distal end 226, a lengthwise axis 227, an intermediate portion 228, an outside diameter 229, a distal portion 230, an exterior surface 231, and a main body 232 that defines a distal tip 234 that is configured to fragment stones disposed within a bodily passage such that they can be removed from the bodily passage.

In the illustrated embodiment, the intermediate portion 228 is disposed between a proximal end of the probe 212 and the distal end 226. The intermediate portion 228 has a first thickness 233 and the distal portion 230 has a second thickness 235 that is greater than the first thickness 233. The second thickness 235 is equal to the outside diameter 229 (e.g., 0.037 inches). The main body 232 defines a curve 236 that transitions between the intermediate portion 228 and the distal portion 230. The distal tip 234 includes four tapered projections 236 that taper along the exterior surface 231 of the probe 212 from a first location between the intermediate portion 228 and the distal end 226 to the distal end 226 and that taper from a second location between the lengthwise axis 227 and the exterior surface 231 to the exterior surface 231.

FIG. 11 illustrates another example of an alternative probe 312 that can be included in a lithotripsy probe assembly. In the illustrated embodiment, the probe 312 has a distal end 326, a lengthwise axis 327, an intermediate portion 328, an outside diameter 329, a distal portion 330, an exterior surface 331, and a main body 332 that defines a distal tip 334 that is configured to fragment stones disposed within a bodily passage such that they can be removed from the bodily passage.

In the illustrated embodiment, the intermediate portion 328 is disposed between a proximal end of the probe 312 and the distal end 326. The outside diameter 329 in this embodiment is equal to about 0.031 inches. The distal tip 334 includes four tapered projections 336 that taper along the exterior surface 331 of the probe 312 from a first location between the intermediate portion 328 and the distal end 326 to the distal end 326 and that taper from a second location between the lengthwise axis 327 and the exterior surface 331 to the exterior surface 331.

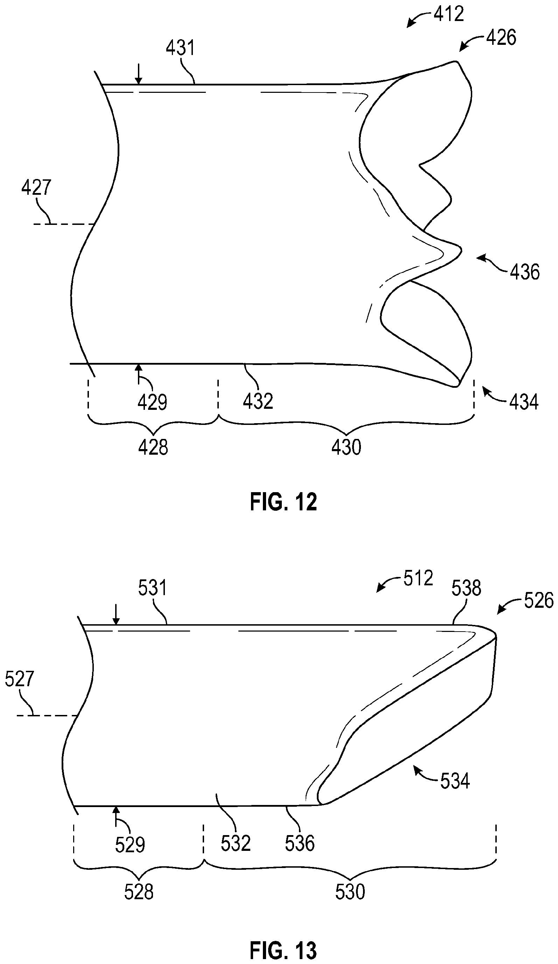

FIG. 12 illustrates another example of an alternative probe 412 that can be included in a lithotripsy probe assembly. In the illustrated embodiment, the probe 412 has a distal end 426, a lengthwise axis 427, an intermediate portion 428, an outside diameter 429, a distal portion 430, an exterior surface 431, and a main body 432 that defines a distal tip 434 that is configured to fragment stones disposed within a bodily passage such that they can be removed from the bodily passage.

In the illustrated embodiment, the intermediate portion 428 is disposed between a proximal end of the probe 412 and the distal end 426. The outside diameter 429 in this embodiment is equal to about 0.027 inches. The distal tip 434 includes a plurality of tapered projections 436 that taper along the exterior surface 431 of the probe 412 from a first location between the intermediate portion 428 and the distal end 426 to the distal end 426 and that taper from a second location between the lengthwise axis 427 and the exterior surface 431 to the exterior surface 431.

FIG. 13 illustrates another example of an alternative probe 512 that can be included in a lithotripsy probe assembly. In the illustrated embodiment, the probe 512 has a distal end 526, a lengthwise axis 527, an intermediate portion 528, an outside diameter 529, a distal portion 530, an exterior surface 531, and a main body 532 that defines a distal tip 534 that is configured to fragment stones disposed within a bodily passage such that they can be removed from the bodily passage.

In the illustrated embodiment, the intermediate portion 528 is disposed between a proximal end of the probe 512 and the distal end 526. The outside diameter 529 in this embodiment is equal to about 0.037 inches. The distal tip 534 tapers from a location between the intermediate portion 528 and the distal end 526 to the distal end 526 and from a first side 536 of the probe 512 toward a second side 538 of the probe 512.

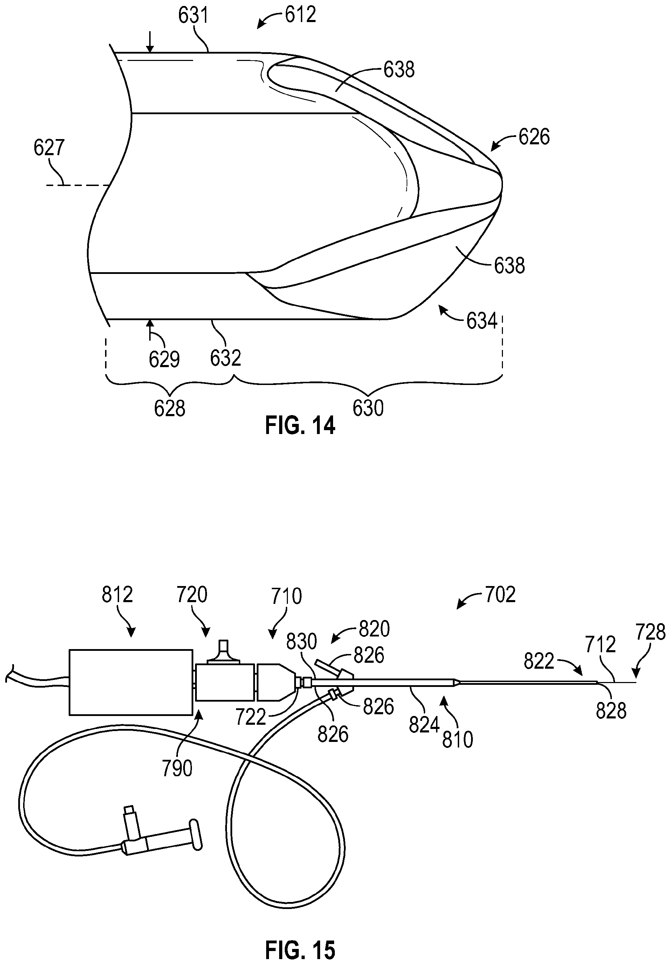

FIG. 14 illustrates another example of an alternative probe 612 that can be included in a lithotripsy probe assembly. In the illustrated embodiment, the probe 612 has a distal end 626, a lengthwise axis 627, an intermediate portion 628, an outside diameter 629, a distal portion 630, an exterior surface 631, and a main body 632 that defines a distal tip 634 that is configured to fragment stones disposed within a bodily passage such that they can be removed from the bodily passage.

In the illustrated embodiment, the intermediate portion 628 is disposed between a proximal end of the probe 612 and the distal end 626. The outside diameter 629 in this embodiment is equal to about 0.031 inches. The distal tip 634 is tapered along the exterior surface 631 of the probe 612 from a location between the intermediate portion 628 and the distal end 626 to the distal end 626 and defines a plurality of recesses 638 that extend into the probe 612. While not included in the embodiments illustrated in FIGS. 11, 12, 13, and 14, a probe can include a cross-sectional configuration along its length similar to that shown in FIG. 10.

FIGS. 15, 16, and 17 illustrate an example lithotripsy system 702 that includes a lithotripsy probe assembly 710, a scope assembly 810, a generator 812, and a suction device 814.

In the illustrated embodiment, the lithotripsy probe assembly 710 is similar to the lithotripsy probe assembly 10 described herein, except as detailed below. The scope assembly 810 is releasably attached to the scope coupling 722 and has a proximal end 820, a distal end 822, and a main body 824 that defines a plurality of ports 826 and a working channel 828. The working channel 828 extends from the proximal end 820 to the distal end 822 and is in fluid communication with a first port 830 of the plurality of ports 826.

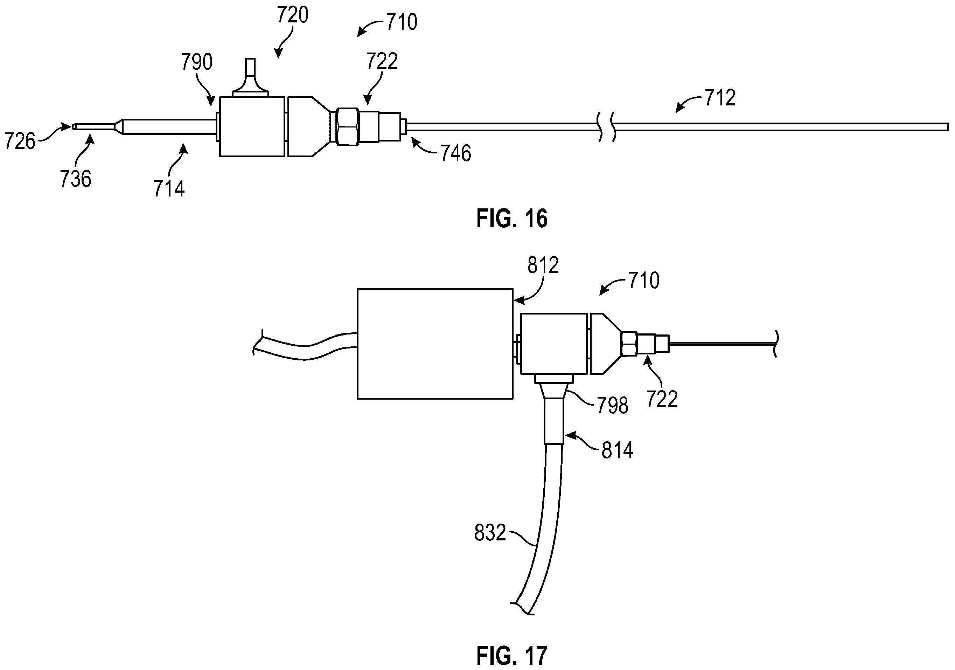

The lithotripsy probe assembly 710 is partially disposed through the scope assembly 810 such that the probe 712 is partially disposed through the working channel 828, the distal end 728 of the probe 712 is disposed distal to the distal end 822 of the scope assembly 810, the scope coupling 722 is attached to the first port 830, and the working channel 828 is in fluid communication with the first passageway 746 of the probe coupling 714. This arrangement provides a mechanism for using the working channel 828 and the lithotripsy probe assembly 710 to remove fluid and/or stone fragments from a bodily passage during treatment. The generator 812 is releasably attached to the proximal end 790 of the housing 720 such that the proximal end 736 of the probe coupling 714 is disposed within the generator 812. The generator 812 is moveable between a deactivated state and an activated state and is configured to transmit energy to the probe 712 (e.g., via the proximal end 726 of the probe 712) and probe coupling 714 (e.g., via the proximal end 736 of the probe coupling) when the generator 812 is in the activated state. The suction device 814, shown in FIG. 17, is shown as a tubular member 832 releasably attached to the side port 798 of the housing 720 such that fluid and fragmented stones can be removed from a bodily passage during treatment.

The lithotripsy probe assemblies described herein can be used in combination with any suitable generator, scope assembly, and suction device and selection of a suitable generator, scope assembly, and suction device to utilize with a lithotripsy probe assembly can be based on various considerations, such as the treatment intended to be performed. Examples of generators considered suitable to utilize with a lithotripsy probe assembly include ballistic lithotripter generators, ultrasonic lithotripter generators, electrohydraulic lithotripsy generators, electrokinetic lithotripsy generators, pneumatic lithotripsy generators, and any other generator considered suitable for a particular embodiment. In the illustrated embodiment, the generator 812 is an electrokinetic lithotripsy generator. Examples of scope assemblies considered suitable to utilize with a lithotripsy probe assembly include ureteroscopes, flexible ureteroscopes, sialendoscopes, and any other scope considered suitable for a particular embodiment. In the illustrated embodiment, the scope assembly 810 is a sialendoscope. Examples of suction devices considered suitable to utilize with a lithotripsy probe assembly include conventional operating room suction devices, variable suction devices, hand-held suction devices, and any other suction device considered suitable for a particular embodiment.

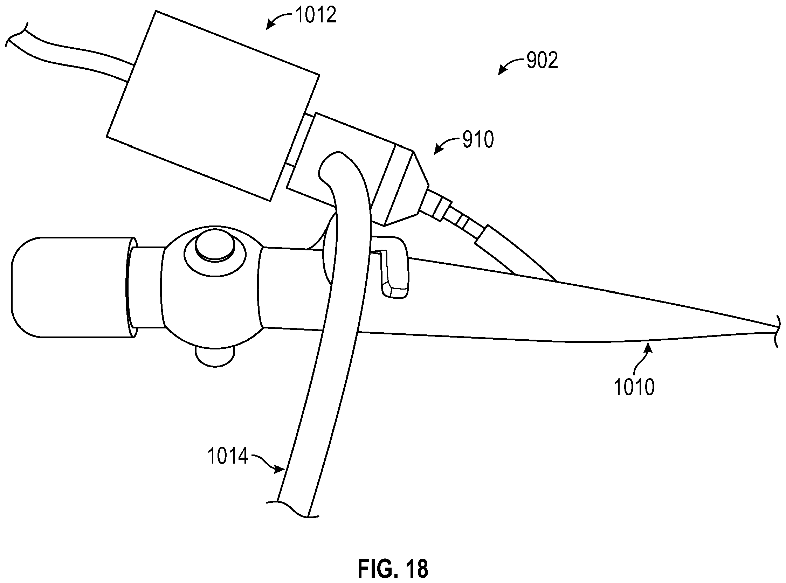

FIG. 18 illustrates another example lithotripsy system 902 that includes a lithotripsy probe assembly 910, a scope assembly 1010, a generator 1012, and a suction device 1014. The lithotripsy system 902 is similar to the lithotripsy system 702 illustrated in FIGS. 15, 16, and 17 and described above. In the illustrated embodiment, the scope assembly 1010 is a flexible ureteroscope and the generator 1012 is an electrokinetic lithotripsy generator.

Various methods of using a lithotripsy system are described herein. While the methods described herein are shown and described as a series of acts, it is to be understood and appreciated that the methods are not limited by the order of acts, as some acts may in accordance with these methods may be omitted, occur in the order shown and/or described, occur in different orders, and/or occur concurrently with other acts described herein.

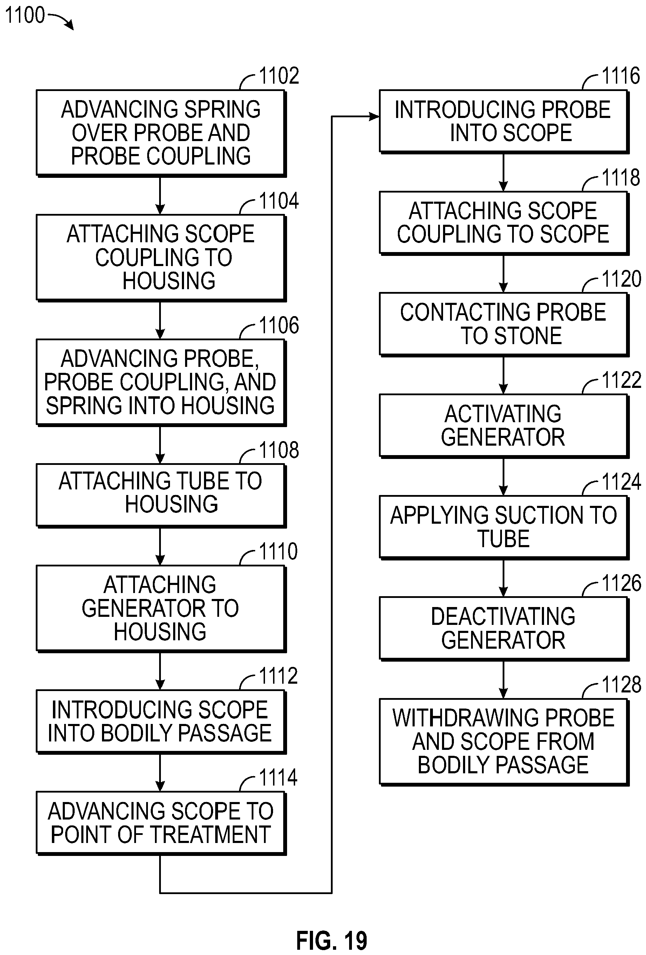

FIG. 19 illustrates a schematic illustration of an example method 1100 of using a lithotripsy system.

An initial step 1102 comprises advancing a spring over a probe and a probe coupling such that the spring contacts a sealing member. Another step 1104 comprising attaching a scope coupling to a housing. Another step 1106 comprises advancing the probe, the probe coupling, and the spring into a first passageway of the housing such that the spring contacts the scope coupling to create a lithotripsy probe assembly. Another step 1108 comprises attaching a first end of a tube to a side port of the housing. Another step 1110 comprises attaching a generator to the housing. Another step 1112 comprises introducing a scope into a bodily passage. Another step 1114 comprises advancing the scope to a point of treatment, such as a stone disposed within the bodily passage. Another step 1116 comprises introducing the probe into a working channel of the scope such that the probe is partially disposed within the working channel and extends distal to the distal end of the scope. Another step 1118 comprises attaching the scope coupling to the scope such that a first passageway of the probe coupling is in fluid communication with the working channel of the scope. Another step 1120 comprises contacting the probe to the stone. Another step 1122 comprises activating the generator such that the probe fragments the stone. Another step 1124 comprises applying suction to the tube such that material disposed outside the working channel of the scope is drawn into the working channel of the scope, through a passageway defined by the scope coupling, through a first passageway of the probe coupling, through a second passageway of the probe coupling, through the side port of the housing, and through the tube. Another step 1126 comprises deactivating the generator. Another step 1128 comprises withdrawing the probe and the scope from the bodily passage.

Step 1102 can be accomplished using any suitable spring, probe, and/or probe coupling, such as those described herein, and such that the spring contacts a sealing member (e.g., third sealing member 78). An optional step that can be completed prior to step 1102 comprises attaching a probe coupling to a probe and can be accomplished by positioning a portion of a probe (e.g., proximal portion) within a first passageway defined by a probe coupling and attaching the probe coupling to the probe using any suitable method or technique of attachment (e.g., press-fitting probe coupling to probe).

Steps 1104 and 1106 can be accomplished using any suitable scope coupling and housing, such as those described herein. Optionally, step 1102, step 1104, and step 1106 can be omitted from method 1100 in embodiments in which a lithotripsy probe assembly is pre-assembled.

Step 1108 can be accomplished using any suitable tube capable of achieving suction and/or irrigation. An optional step comprises attaching a second end of the tube to a suction device and/or irrigation device.

Step 1110 can be accomplished using any suitable generator. Examples of generators considered suitable to attach to a housing include ballistic lithotripter generators, ultrasonic lithotripter generators, electrohydraulic lithotripsy generators, electrokinetic lithotripsy generators, pneumatic lithotripsy generators, and any other generator considered suitable for a particular embodiment.

Step 1112 can be accomplished using any suitable scope, such as those described herein, and by applying a distally-directed force on the scope such that it is advanced into a bodily passage. Examples of bodily passages within which it is considered suitable to advance a scope include a portion of the urinary tract, a portion of a salivary duct, a portion of a surgically-created bodily passage, and any other bodily passage considered suitable for a particular embodiment.

Step 1114 can be accomplished by applying a force (e.g., distally-directed, proximally-directed, torque) on the scope until it is advanced to a point of treatment within the bodily passage.

Step 1116 can be accomplished such that any suitable length of the probe is disposed distal to the distal end of the scope. Examples of lengths of a probe considered suitable to position distal to a distal end of a scope include lengths equal to, greater than, less than, or about 7 millimeters, 7.5 millimeters, 8 millimeters, 8.5 millimeters, 9 millimeters, 9.5 millimeters, 10 millimeters, between 5 and 12 millimeters, and any other length considered suitable for a particular embodiment. For example, in embodiments in which ureteroscopy is being performed a probe can extend 7 millimeters or 7.5 millimeters distal to a distal end of a scope and in embodiments in which sialendoscopy is being performed a probe can extend from between 8 millimeters and 10 millimeters distal to a distal end of a scope.

Step 1118 can be accomplished by applying a torque to the scope coupling such that it becomes releasably attached to the scope. In alternative embodiments, step 1116 and step 1118 can be accomplished prior to step 1112.

Step 1120 can be accomplished by applying a force (e.g., distally-directed, proximally-directed, torque) on a portion of the lithotripsy probe assembly and/or scope until the probe contacts the stone.

Step 1122 can be accomplished by manipulating the position of the lithotripsy probe assembly and/or scope (e.g., applying a proximally-directed force to the lithotripsy probe assembly and/or scope, applying a distally-directed force to the lithotripsy probe assembly and/or scope, and/or applying torque to the lithotripsy probe assembly and/or scope) while the generator is in the activated state such that the distal tip of the probe fragments the stone.

Step 1124 can be accomplished by activating a suction device such that fragmented stone can be withdrawn from the bodily passage. Alternatively, irrigation can be provided through the scope (e.g., through the tube attached to the housing or through a second tube attached to the scope). Optionally, irrigation and suction can be accomplished simultaneously. Step 1124, and its alternative and optional variations, can optionally be accomplished concurrently with step 1122.

Step 1128 can be accomplished by applying a proximally-directed force on the lithotripsy probe assembly and/or scope such that they are withdrawn from the bodily passage.

Any of the steps described in method 1100 can be repeated any suitable number of times. The lithotripsy probe assemblies, lithotripsy systems, and methods described herein can be utilized for urologic stone management, otolaryngology stone management, percutaneous nephrolithotomy (PNCL), flexible ureteroscopy (URS), endoscopy, and sialendoscopy. For example, the lithotripsy probe assemblies and lithotripsy systems described herein provide both capability with flexible ureteroscopes and sialendoscopes and the provision of suction for debris clearance to maintain a clear field of view. By forming the probes described herein as a solid member that partially extends through a probe coupling that is utilized for irrigation and/or suction, the probes described herein can be used to treat a greater number of conditions and bodily passages since they have a reduced outside diameter and increased flexibility relative to probes that define passageways through their length, or a portion of their length.

In view of the foregoing it will be appreciated that one aspect of the disclosure provides a lithotripsy probe assembly comprising: a probe coupling having a probe coupling lengthwise axis, a probe coupling proximal end, a probe coupling distal end, a probe coupling length, and a probe coupling main body defining a probe coupling side wall, a plurality of projections, a probe coupling first passageway, and a probe coupling second passageway, the probe coupling length extending from the probe coupling proximal end to the probe coupling distal end, each projection of the plurality of projections extending from the probe coupling side wall and away from the probe coupling lengthwise axis, the probe coupling first passageway extending from the probe coupling proximal end to the probe coupling distal end, the probe coupling second passageway extending through the probe coupling side wall and in fluid communication with the probe coupling first passageway; a probe attached to the probe coupling and partially disposed within the probe coupling first passageway, the probe having a probe proximal end, a probe distal end, a probe length, and a probe main body defining a probe distal tip, the probe length extending from the probe proximal end to the probe distal end, the probe length greater than the probe coupling length; a plurality of sealing members disposed on the probe coupling, a sealing member of the plurality of sealing members disposed between the plurality of projections and the probe coupling distal end; a spring disposed on the probe coupling and disposed adjacent to the sealing member of the plurality of sealing members; and a housing disposed on the probe, the probe coupling, and the spring, the housing having a housing proximal end, a housing distal end, and a housing main body defining a housing side wall, a housing side port, a housing first passageway, and a housing second passageway, the housing first passageway extending from the housing proximal end to the housing distal end, the housing second passageway extending through the housing side port and in fluid communication with the housing first passageway and the probe coupling second passageway.

Another aspect provides a lithotripsy probe assembly comprising: a probe coupling having a probe coupling lengthwise axis, a probe coupling proximal end, a probe coupling distal end, a probe coupling length, and a probe coupling main body defining a probe coupling side wall, a plurality of projections, a probe coupling first passageway, and a probe coupling second passageway, the probe coupling length extending from the probe coupling proximal end to the probe coupling distal end, each projection of the plurality of projections extending from the probe coupling side wall and away from the probe coupling lengthwise axis, the probe coupling first passageway extending from the probe coupling proximal end to the probe coupling distal end, the probe coupling second passageway extending through the probe coupling side wall and in fluid communication with the probe coupling first passageway; a probe attached to the probe coupling and partially disposed within the probe coupling first passageway, the probe having a probe proximal end, a probe distal end, a probe length, and a probe main body defining a probe distal tip, the probe length extending from the probe proximal end to the probe distal end, the probe length greater than the probe coupling length; a plurality of sealing members disposed on the probe coupling, a sealing member of the plurality of sealing members disposed between the plurality of projections and the probe coupling distal end; a spring disposed on the probe coupling and contacting the sealing member of the plurality of sealing members; a housing disposed on the probe, the probe coupling, and the spring, the housing having a housing proximal end, a housing distal end, and a housing main body defining a housing side wall, a housing side port, a housing first passageway, and a housing second passageway, the housing first passageway extending from the housing proximal end to the housing distal end, the housing second passageway extending through the housing side port and in fluid communication with the housing first passageway and the probe coupling second passageway; and a scope coupling releasably attached to the housing and disposed adjacent to the spring.

Those with ordinary skill in the art will appreciate that various modifications and alternatives for the described and illustrated examples can be developed in light of the overall teachings of the disclosure, and that the various elements and features of one example described and illustrated herein can be combined with various elements and features of another example without departing from the scope of the invention. Accordingly, the particular arrangement of elements and steps disclosed herein have been selected by the inventor(s) simply to describe and illustrate examples of the invention and are not intended to limit the scope of the invention or its protection, which is to be given the full breadth of the appended claims and any and all equivalents thereof.

## Claims

1. A lithotripsy probe assembly comprising:
a probe coupling having a probe coupling lengthwise axis, a probe coupling proximal end, a probe coupling distal end, a probe coupling length, and a probe coupling main body defining a probe coupling side wall, a plurality of projections, a probe coupling first passageway, and a probe coupling second passageway, the probe coupling length extending from the probe coupling proximal end to the probe coupling distal end, each projection of the plurality of projections extending from the probe coupling side wall and away from the probe coupling lengthwise axis, the probe coupling first passageway extending from the probe coupling proximal end to the probe coupling distal end, the probe coupling second passageway extending through the probe coupling side wall and in fluid communication with the probe coupling first passageway; and
a probe attached to the probe coupling and partially disposed within the probe coupling first passageway, the probe having a probe proximal end, a probe distal end, a probe length, and a probe main body defining a probe distal tip, the probe length extending from the probe proximal end to the probe distal end, the probe length greater than the probe coupling length.

2. The lithotripsy probe assembly of claim 1, wherein the probe coupling first passageway has an inside diameter; and
wherein the probe has an outside diameter that is less than the inside diameter of the probe coupling first passageway.

3. The lithotripsy probe assembly of claim 1, wherein the probe coupling first passageway has a first inside diameter and a second inside diameter that is greater than the first inside diameter; and
wherein the probe has an outside diameter that is less than the second inside diameter of the probe coupling first passageway.

4. The lithotripsy probe assembly of any preceding claim, further comprising a plurality of sealing members disposed on the probe coupling; and
further comprising a spring disposed on the probe coupling and disposed adjacent to a sealing member of the plurality of sealing members, for example wherein the spring contacts the sealing member of the plurality of sealing members.

5. The lithotripsy probe assembly of claim 4, wherein the probe coupling has a distal portion extending from the plurality of projections to the probe coupling distal end, the distal portion having a distal portion length; and
wherein the spring has a spring length that is greater than the distal portion length.

6. The lithotripsy probe assembly of claim 4 or 5, further comprising a housing disposed on the probe, the probe coupling, and the spring, the housing having a housing proximal end, a housing distal end, and a housing main body defining a housing side wall, a housing side port, a housing first passageway, and a housing second passageway, the housing first passageway extending from the housing proximal end to the housing distal end, the housing second passageway extending through the housing side port and in fluid communication with the housing first passageway and the probe coupling second passageway,
for example further comprising a scope coupling releasably attached to the housing and disposed adjacent to the spring, for example wherein the scope coupling contacts the spring.

7. The lithotripsy probe assembly of any preceding claim, wherein the probe has a proximal portion extending from the probe proximal end toward the probe distal end; and
wherein the probe coupling is attached to the proximal portion of the probe.

8. The lithotripsy probe assembly of any preceding claim, wherein a first set of projections of the plurality of projections comprises a first projection and a second projection; and
wherein a first sealing member is disposed between the first projection of the first set of projections and the second projection of the first set of projections, for example wherein a second set of projections of the plurality of projections comprises a third projection and a fourth projection; and
wherein a second sealing member is disposed between the first projection of the second set of projections and the second projection of the second set of projections.

9. The lithotripsy probe assembly of claim 8, wherein a third sealing member is disposed between the second set of projections and the probe coupling distal end.

10. The lithotripsy probe assembly of any preceding claim, wherein the distal tip defines at least one of: (a) a plurality of tapered projections; and (b) a plurality of recesses.

11. The lithotripsy probe assembly of any preceding claim, wherein the probe is formed of a first material and the probe coupling is formed of a second material that is different than the first material.

12. The lithotripsy probe assembly of any preceding claim wherein the probe is formed of Nitinol.

13. The lithotripsy probe assembly of any preceding claim wherein the probe distal end is disposed within the probe coupling first passageway.

14. The lithotripsy probe assembly of claim 1 comprising:
a plurality of sealing members disposed on the probe coupling, a sealing member of the plurality of sealing members disposed between the plurality of projections and the probe coupling distal end;
a spring disposed on the probe coupling and disposed adjacent to the sealing member of the plurality of sealing members; and
a housing disposed on the probe, the probe coupling, and the spring, the housing having a housing proximal end, a housing distal end, and a housing main body defining a housing side wall, a housing side port, a housing first passageway, and a housing second passageway, the housing first passageway extending from the housing proximal end to the housing distal end, the housing second passageway extending through the housing side port and in fluid communication with the housing first passageway and the probe coupling second passageway.

15. The lithotripsy probe assembly of claim 1 comprising:
a plurality of sealing members disposed on the probe coupling, a sealing member of the plurality of sealing members disposed between the plurality of projections and the probe coupling distal end;
a spring disposed on the probe coupling and contacting the sealing member of the plurality of sealing members;
a housing disposed on the probe, the probe coupling, and the spring, the housing having a housing proximal end, a housing distal end, and a housing main body defining a housing side wall, a housing side port, a housing first passageway, and a housing second passageway, the housing first passageway extending from the housing proximal end to the housing distal end, the housing second passageway extending through the housing side port and in fluid communication with the housing first passageway and the probe coupling second passageway; and
a scope coupling releasably attached to the housing and disposed adjacent to the spring.
